# EUROPÄISCHE PATENTANMELDUNG

(11) **EP 4 813 974 A1**
(43) Veröffentlichungstag der Anmeldung: **30.09.2026**
(21) Anmeldenummer: 26165408.1
(22) Anmeldetag: 17.03.2026
(51) Int. Cl.: C07D 281/06

(54) **SCHWEFELHALTIGES LACTAM UND VERFAHREN ZU DESSEN HERSTELLUNG**

(30) Priorität: 26.03.2025 DE 102025111680
(71) Anmelder: Rheinisch-Westfälische Technische Hochschule Aachen, abgekürzt RWTH Aachen, Körperschaft des öffentlichen Rechts, 52062 Aachen (DE)
(72) Erfinder: Palkovits, Regina, 52074 Aachen (DE); Sebers, Rebecca, 52070 Aachen (DE); Hausoul, Peter J.C., 6372 NR Landgraaf (NL)
(74) Vertreter: Michalski Hüttermann & Partner mbB

(57) **Zusammenfassung**

Die Erfindung betrifft ein schwefelhaltiges Lactam gemäß der allgemeinen Formel (1), insbesondere ein schwefelhaltiges Caprolactam, sowie ein Verfahren zu dessen Herstellung durch Umsetzen eines Dialkylmaleats gemäß der allgemeinen Formel (2) mit einem Aminoalkylthiol gemäß der allgemeinen Formel (3).

## Beschreibung

Die Erfindung betrifft ein schwefelhaltiges Lactam, insbesondere ein schwefelhaltiges Caprolactam, und ein Verfahren zu dessen Herstellung.

Eine allmähliche Erschöpfung der nicht erneuerbaren fossilen Ressourcen und das zunehmende Problem der Umweltverschmutzung machen es essenziell, die Abhängigkeit der chemischen Industrie von fossilen Ressourcen zu verringern und Plattformchemikalien auf Basis nachwachsender Rohstoffe zur Verfügung zu stellen, um einen Wandel zu einer nachhaltigen chemischen Industrie zu stützen.

Eine bedeutende Plattformchemikalie ist Caprolactam und dessen Derivate. Caprolactam wird insbesondere für die Herstellung von Fasern aus Polyamid 6 verwendet. S. Zhang et al., Polymers 2019, 11, 978, beschreiben beispielsweise die Verwendung von Caprolactam in der Herstellung von Nylon. N. Cortez-Lemus, A. Licea-Claverie, Progress in Polymer Science, Vol. 53, 2016, 1-51, beschreiben das thermoresponsive Polymer Poly(N-vinylcaprolactam), dessen Herstellung und Verwendung. Die Anwendungsbereiche von Caprolactam und dessen Derivaten sind vielfältig. CN 113999206 A beschreibt ein Isoquinolin-1,3-diamin-Derivat von Caprolactam für pharmazeutische Anwendungen.

Insbesondere schwefelhaltige Derivate von Caprolactam sind vielfältig verwendbar. G. Wang, et al., Chem. Eur. J. 2017, 23, 554-557, beschreiben die enantioselektive Synthese von NHfreien 1,5-Benzothiazepinen und eine pharmakologische Anwendbarkeit schwefelhaltiger Caprolactam-Derivate. US 2014/0206095 A1 beschreibt die Reaktion von Cystein mit einem Acrylat zu einem Thioether, der unter intramolekularer Zyklisierung zu zyklischem 3-Carboxy-5-oxoperhydro-1,4-thiazepin reagiert, sowie eine Reaktion mit Homocystein zum analogen achtgliedrigen Ring. US 2012/282386 A1 beschreibt essbare Salze von schwefelhaltigen Caprolactam-Derivaten und deren Verwendung als Aromaverstärker. JP 2014073979 A beschreibt Triazolothiazepin-Derivate und deren Verwendung als Wirkstoff gegen Diabetes. Es besteht daher ein Bedarf an weiteren schwefelhaltigen Caprolactamen.

Der vorliegenden Erfindung lag die Aufgabe zu Grunde, schwefelhaltige Caprolactame und ein Verfahren zu deren Herstellung zur Verfügung zu stellen.

Diese Aufgabe wird gelöst durch ein schwefelhaltiges Lactam insbesondere schwefelhaltiges Caprolactam gemäß Anspruch 1 sowie ein Verfahren zur Herstellung des schwefelhaltigen Lactams, insbesondere schwefelhaltigen Caprolactams, gemäß Anspruch 3. Bevorzugte Ausgestaltungen der Erfindung sind in den Unteransprüchen und der Beschreibung offenbart, wobei weitere in den Unteransprüchen oder in der Beschreibung beschriebene Merkmale einzeln oder in einer beliebigen Kombination einen Gegenstand der Erfindung darstellen können, wenn sich aus dem Kontext nicht eindeutig das Gegenteil ergibt.

Die vorliegende Erfindung betrifft ein schwefelhaltiges Lactam gemäß der nachstehenden allgemeinen Formel (1): 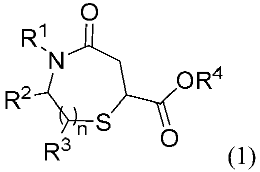 worin:
- R¹, R⁴: sind unabhängig voneinander ausgewählt aus der Gruppe umfassend Wasserstoff und C₁-C₃-Alkyl,
- R², R³: sind unabhängig voneinander ausgewählt aus der Gruppe umfassend Wasserstoff, C₁-C₃-Alkyl, COOH und COOMe,
- n: ist 1 oder 2.

Es wird ein schwefelhaltiges Lactam, insbesondere ein schwefelhaltiges Caproclactam, zur Verfügung gestellt, das durch den Schwefel im Lactamzyklus und eine Carboxyl- oder Esterseitenkette eine vorteilhafte Kombination von Funktionalitäten in einem chemischen Baustein ermöglicht.

Der Begriff "C₁-C₃-Alkyl" umfasst, wenn nicht anders angegeben, geradkettige oder verzweigte Alkylgruppen mit 1 bis 3 Kohlenstoffatomen. C₁-C₃-Alkyl-Gruppen sind insbesondere ausgewählt aus der Gruppe umfassend Methyl, Ethyl, Propyl und Isopropyl.

Der Begriff "Lactam" bezeichnet cyclische Verbindungen, die eine Amidbindung im Ring enthalten. Schwefelhaltige Lactame gemäß der allgemeinen Formel (1) mit n ist 1 oder 2 umfassen sieben- und achtgliedrige Ringe. Der Begriff "Lactam" umfasst im Sinn der vorliegenden Anmeldung entsprechend sieben- und achtgliedrige Lactame. Siebengliedrige Lactame werden auch als ε-Lactame bezeichnet, achtgliedrige Lactame als ζ-Lactame.

R², R³ können unabhängig voneinander ausgewählt sein aus der Gruppe umfassend Wasserstoff, C₁-C₃-Alkyl, COOH und COOMe, wobei "Me" Methyl bedeutet. Vorzugsweise sind R² und R³ unabhängig voneinander ausgewählt aus der Gruppe umfassend Wasserstoff, Methyl und Ethyl. In Ausführungsformen sind R¹, R², R³ und R⁴ unabhängig voneinander ausgewählt aus der Gruppe umfassend Wasserstoff, Methyl und Ethyl, wobei n 1 oder 2 sein kann. In Ausführungsformen sind R¹, R² und R³ unabhängig voneinander ausgewählt aus der Gruppe umfassend Wasserstoff, Methyl und Ethyl, und R⁴ ist ausgewählt aus Methyl und Ethyl, wobei n 1 oder 2 sein kann. In bevorzugten Ausführungsformen ist R¹ ausgewählt aus der Gruppe umfassend Wasserstoff, Methyl und Ethyl, R² und R³ sind Wasserstoff, und R⁴ ist ausgewählt aus Methyl und Ethyl, wobei n gleich 1 oder 2 sein kann. In auch bevorzugten Ausführungsformen ist R¹ Wasserstoff, R² und R³ sind Wasserstoff, und R⁴ ist ausgewählt aus Methyl und Ethyl, insbesondere Methyl, wobei n gleich 1 oder 2 sein kann.

In bevorzugten Ausführungsformen ist n gleich 1 und die Verbindung ist ein schwefelhaltiges Caprolactam. In Ausführungsformen ist n gleich 1 und R¹, R², R³ und R⁴ sind unabhängig voneinander ausgewählt aus der Gruppe umfassend Wasserstoff, Methyl und Ethyl. In Ausführungsformen ist n gleich 1 und R¹, R² und R³ sind unabhängig voneinander ausgewählt aus der Gruppe umfassend Wasserstoff, Methyl und Ethyl, und R⁴ ist ausgewählt aus Methyl und Ethyl. In bevorzugten Ausführungsformen ist n gleich 1, R¹ ist ausgewählt aus der Gruppe umfassend Wasserstoff, Methyl und Ethyl, R² und R³ sind Wasserstoff, und R⁴ ist ausgewählt aus Methyl und Ethyl. In auch bevorzugten Ausführungsformen ist R¹ Wasserstoff, R² und R³ sind Wasserstoff, und R⁴ ist ausgewählt aus Methyl und Ethyl, insbesondere Methyl, wobei n gleich 1 oder 2 sein kann.

In bevorzugten Ausführungsformen ist das schwefelhaltige Lactam ein schwefelhaltiges Caprolactam gemäß der nachstehenden Formel (4):

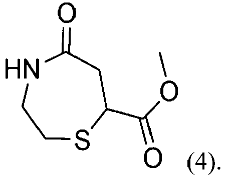

Schwefelhaltige Lactame der allgemeinen Formel (1) wie vorstehend beschrieben sind insbesondere gemäß dem nachstehend beschriebenen Verfahren herstellbar.

Die vorliegende Erfindung betrifft ferner ein Verfahren zur Herstellung von schwefelhaltigen Lactamen, gemäß der allgemeinen Formel (1), insbesondere schwefelhaltigen Caprolactamen, wobei man ein Dialkylmaleat gemäß der allgemeinen Formel (2) mit einem Aminoalkylthiol gemäß der allgemeinen Formel (3) umsetzt: 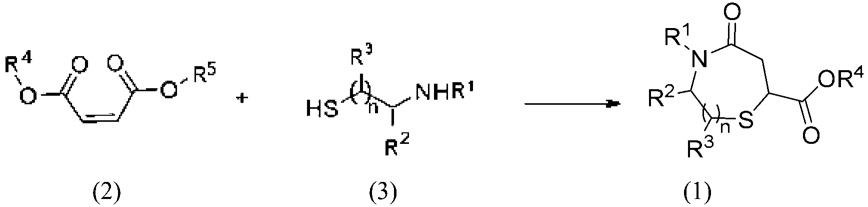 worin:
R¹, R⁴, R⁵ sind unabhängig voneinander ausgewählt aus der Gruppe umfassend Wasserstoff und C₁-C₃-Alkyl,
R², R³ sind unabhängig voneinander ausgewählt aus der Gruppe umfassend Wasserstoff, C₁-C₃-Alkyl, COOH und COOMe,
n ist 1 oder 2.

Das erfindungsgemäße Verfahren erlaubt die Synthese schwefelhaltiger Lactame, insbesondere schwefelhaltiger Caproclactam-Derivate aus nachwachsenden Rohstoffen auf Biomassebasis. Die Herstellung kann aus nachwachsenden Rohstoffen erfolgen und nicht nur im Hinblick auf eine wirtschaftliche Rohstoffnutzung, sondern auch im Hinblick auf die Reaktionsführung in nachhaltiger Weise erfolgen. Schwefelhaltige Lactame, insbesondere schwefelhaltige Caproclactam-Derivate können dabei in sehr guter Produktrohausbeute bei Umgebungstemperatur erhalten werden.

In Ausführungsformen sind R⁴ und R⁵ unabhängig voneinander ausgewählt aus der Gruppe umfassend C₁-C₃-Alkyl. C₁-C₃-Alkylgruppen bilden gute Abgangsgruppen. In Ausführungsformen ist das Dialkylmaleat gemäß der allgemeinen Formel (2) ausgewählt aus der Gruppe umfassend Dimethylmaleat und Diethylmaleat, insbesondere Dimethylmaleat. Sowohl Ethyl- wie auch Methylsubstituenten stellen gute Abgangsgruppen für die Reaktion zur Verfügung. Das Dialkylmaleat kann bezogen auf das Aminoalkylthiol in stöchiometrischen Mengen verwendet werden.

In Ausführungsformen sind R¹, R² und R³ unabhängig voneinander ausgewählt aus der Gruppe umfassend Wasserstoff, Methyl und Ethyl. In Ausführungsformen ist R¹ ausgewählt aus der Gruppe umfassend Wasserstoff, Methyl und Ethyl und R² und R³ sind Wasserstoff. In Ausführungsformen sind R¹, R² und R³ Wasserstoff. In Ausführungsformen ist das Aminoalkylthiol gemäß der allgemeinen Formel (3) ausgewählt aus der Gruppe umfassend 2-Aminoethanthiol (Cysteamin) und 3-Aminopropanthiol. 3-Aminopropanthiol erlaubt die Herstellung von achtgliedrigen Lactamen. 2-Aminoethanthiol, auch Cysteamin genannt, erlaubt die Herstellung schwefelhaltiger Caproclactam-Derivate. Insbesondere Cysteamin erlaubt eine biobasierte Herstellung schwefelhaltiger Caproclactam-Derivate. Das Aminoalkylthiol, insbesondere 2-Aminoethanthiol und 3-Aminopropanthiol, wird vorzugsweise in Form seines Hydrochlorids verwendet, da das freie Thiol leicht zum korrespondierenden Disulfid oxidiert wird.

Vorzugsweise führt man die Umsetzung unter Zugabe einer Base wie Triethylamin, Diisopropylethylamin (DIPEA), 2,6-Lutidin oder Pyridin durch. In Ausführungsformen führt man die Umsetzung unter Zugabe von Triethylamin durch. Das Triethylamin kann bezogen auf das Dialkylmaleat bzw. das Aminoalkylthiol in stöchiometrischen Mengen oder vorzugsweise im Überschuss verwendet werden. Vorzugsweise führt man die Umsetzung mit einem Überschuss an Triethylamin im Bereich von ≥ 1,2 eq. bis ≤ 1,5 eq. durch. Bei einem derartigen leichten Überschuss konnten gute Ausbeuten erzielt werden.

In Ausführungsformen führt man die Umsetzung in einem Lösemittel ausgewählt aus der Gruppe umfassend Wasser, Alkohole, Dioxane, Acetonitril, Ether, Furan, Tetrahydrofuran, oder deren Gemischen durch. Vorzugsweise führt man die Umsetzung in einem Alkohol als Lösemittel durch. Der Begriff "Alkohol" umfasst hierbei einwertige und mehrwertige, insbesondere zweiwertige Alkohole. Bevorzugt ist der Alkohol ausgewählt aus der Gruppe umfassend Methanol, Ethanol, Propanol, Isopropanol, Butanol, tert.-Butanol und/oder Ethylenglykol. Vorzugsweise ist der Alkohol ausgewählt aus der Gruppe umfassend Methanol und Ethanol. Eine Umsetzung in Methanol oder Ethanol ist bevorzugt und erbrachte sehr hohe Ausbeuten. Der Begriff "Dioxane" umfasst 1,3-Dioxan und 1,4-Dioxan. Eine Umsetzung in Dioxan ist ebenfalls bevorzugt und erbrachte eine sehr hohe Ausbeute. Ebenfalls bevorzugt ist eine Umsetzung in Wasser, vorzugsweise vollentsalztes Wasser, oder wässriger Lösung. Auch in Wasser wurden gute Ausbeuten erzielt. Ebenfalls bevorzugt ist eine Umsetzung in Acetonitril. Auch in Acetonitril wurden verwendbare Ausbeuten erzielt.

Das Lösemittel kann auch ein Gemisch von wenigstens zwei Lösemitteln ausgewählt aus Wasser, Alkohole, Dioxane, Acetonitril, Ether, Furan und Tetrahydrofuran sein. Insbesondere wässrige Gemische von Wasser mit einem oder mehreren organischen Lösemitteln beispielsweise ausgewählt aus Alkoholen, Dioxane, Acetonitril, Furan oder Tetrahydrofuran sind verwendbar. Bevorzugt sind insbesondere Gemische von Wasser und Alkohol, beispielsweise Wasser und Ethanol oder Methanol.

In Ausführungsformen führt man die Umsetzung bei einer Temperatur im Bereich von ≥ 5 °C bis ≤ 95 °C, vorzugsweise bei einer Temperatur im Bereich von ≥ 15 °C bis ≤ 25 °C, durch. In diesen Temperaturbereichen konnten gute Ausbeuten erhalten werden. Insbesondere kann die Reaktion bei Umgebungstemperatur, beispielsweise 19-22 °C, durchgeführt werden, wodurch ohne energetischen Aufwand für Kühlung oder Erhitzung eine hohe Wirtschaftlichkeit erzielt werden kann.

Die Reaktionszeit der Umsetzung kann im Bereich von ≥ 5 Minuten bis ≤ 24 Stunden, bevorzugt im Bereich von ≥ 30 Minuten bis ≤ 24 Stunden, liegen. Grundsätzlich hängt die Reaktionszeit von der Reaktivität des Substrats ab. Vorteilhafterweise wurden keine offensichtlichen Nebenreaktionen beobachtet, so dass nach Ablauf der Reaktion das Reaktionsgemisch stabil blieb. Das Verfahren kann grundsätzlich kontinuierlich oder diskontinuierlich, beispielsweise in Rührkesseln, Synthesereaktoren wie Rührreaktoren oder Autoklaven durchgeführt werden. Es ist von Vorteil, dass die Umsetzung nicht notwendigerweise unter Stickstoffatmosphäre durchgeführt werden muss.

Die aus dem Verfahren resultierenden Reaktionsgemische können nach herkömmlichen Methoden aufgereinigt werden, beispielsweise thermisch mittels destillativer oder rektifikativer Verfahren. In bevorzugten Ausführungsformen reinigt man das schwefelhaltige Lactam, insbesondere schwefelhaltige Caprolactam, mittels Extraktion mit Diethylether, insbesondere bei einer Temperatur im Bereich von ≥ 25 °C bis ≤ 40 °C, auf. Durch eine Extraktion mit leicht erwärmtem Diethylether konnte schwefelhaltiges Caprolactam in Form reiner weißer Kristalle erhalten werden.

Insgesamt kann ein Verfahren zur Verfügung gestellt werden, das unter Verwendung von gut verfügbaren, ressourcenschonenden Edukten eine Herstellung schwefelhaltiger Lactame, insbesondere schwefelhaltigen Caprolactams, in einer einschrittigen Umsetzung in guter Ausbeute erlaubt.

Ein weiterer Gegenstand betrifft ein schwefelhaltiges Lactam gemäß der allgemeinen Formel (1) wie vorstehend beschrieben, insbesondere ein schwefelhaltiges Caprolactam, vorzugsweise gemäß Formel (4), hergestellt nach dem hierin beschriebenen Verfahren. Für die Beschreibung des schwefelhaltigen Lactams wie auch des Verfahrens wird auf die vorstehende Beschreibung Bezug genommen.

Wenn nicht anders ausgeführt, weisen die verwendeten technischen und wissenschaftlichen Ausdrücke die Bedeutung auf, wie sie gemeinhin von einem Durchschnittsfachmann in dem Gebiet, zu dem diese Erfindung gehört, verstanden wird.

Beispiele, die der Veranschaulichung der vorliegenden Erfindung dienen, sind nachstehend angegeben.

### Chemikalien:

Die verwendeten Edukte wurden von Sigma-Aldrich, Fluorochem, Aldrich oder abcr bezogen. Lösemittel wurden von Chemsolute GmbH bezogen.

### Kernspinresonanz (NMR):

Die Reaktionsprodukte wurden mittels ¹H-NMR und mittels ¹³C-NMR mithilfe eines Bruker DPX-400 FT-NMR-Spektrometer bei 400 MHz und Raumtemperatur gemessen. Die Signale und die chemische Verschiebung in δ-Einheiten wurden auf die restlichen Protonensignale des deuterierten Lösungsmittels bezogen 1H: DMSO-d6 (δH = 2,50 ppm); DMSO-d6 (δC = 39,52 ppm). Unter Verwendung von Mesitylen als Standard wurde quantitativ analysiert.

### Beispiel 1

### Umsetzung von Dimethylmaleat mit Cysteaminhydrochlorid zu schwefelhaltigem Caprolactam gemäß Formel (4)

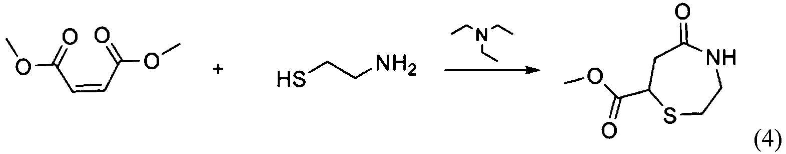

Dimethylmaleat (5 mL, 40 mmol, 1 eq) und Cysteaminhydrochlorid (4,544 g, 40 mmol) wurden bei Umgebungstemperatur (20±2 °C) zu 40 mL Methanol (MeOH) gegeben. Anschließend wurden langsam 7 mL Trietylamin (1.2 eq) zugegeben. Die Mischung wurde für 30 Minuten gerührt. Im Anschluss wurde das Lösemittel durch Rotation entfernt, bevor das Produkt mit leicht erwärmten Diethylether (40 mL) unter Rühren für 5 Minuten und Filtrieren verbleibender Feststoffe aufgereinigt wurde. Das Filtrat wurde bei 8 °C gekühlt und das Produkt wurde in Form weißer Kristalle erhalten und mittels ¹H-NMR und ¹³C-NMR bestätigt.

### Beispiel 2

### Bestimmung der Ausbeute der Umsetzung von Dimethylmaleat mit Cysteaminhydrochlorid in verschiedenen Lösemitteln

Die Umsetzung von Dimethylmaleat mit Cysteaminhydrochlorid wurde wie in Beispiel 1 beschrieben, jedoch unter Verwendung verschiedener Lösemittel wiederholt. Die folgende Tabelle 1 fasst die erhaltenen Rohausbeuten, ermittelt mittels ¹H-NMR zusammen.

**Tabelle 1: Rohausbeuten der Umsetzung von Dimethylmaleat mit Cysteaminhydrochlorid**

| Lösemittel | Ausbeute [%] |
|---|---|
| Methanol | 99 |
| Ethanol | 89 |
| Wasser (vollentsalzt) | 76 |
| Dioxan | 99 |
| Ethylacetat | 0 |
| Acetonitril | 45 |

Die Ergebnisse zeigen insgesamt, dass eine Herstellung von schwefelhaltigem Caprolactam aus der Umsetzung von Dimethylmaleat mit Cysteaminhydrochlorid in einer einschrittigen Reaktion mit guter Ausbeute zur Verfügung gestellt werden kann.

## Patentansprüche

1. Schwefelhaltiges Lactam gemäß der nachstehenden allgemeinen Formel (1): 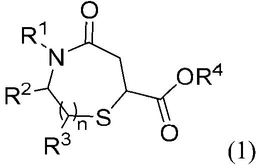 worin:
R¹, R⁴ sind unabhängig voneinander ausgewählt aus der Gruppe umfassend Wasserstoff und C₁-C₃-Alkyl,
R², R³ sind unabhängig voneinander ausgewählt aus der Gruppe umfassend Wasserstoff, C₁-C₃-Alkyl, COOH und COOMe,
n ist 1 oder 2.

2. Schwefelhaltiges Lactam nach Anspruch 1, **dadurch gekennzeichnet, dass** das Lactam ein schwefelhaltiges Caprolactam gemäß der nachstehenden Formel (4) ist:

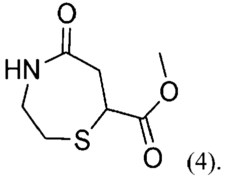

3. Verfahren zur Herstellung von schwefelhaltigen Lactamen gemäß der allgemeinen Formel (1), wobei man ein Dialkylmaleat gemäß der allgemeinen Formel (2) mit einem Aminoalkylthiol gemäß der allgemeinen Formel (3) umsetzt: 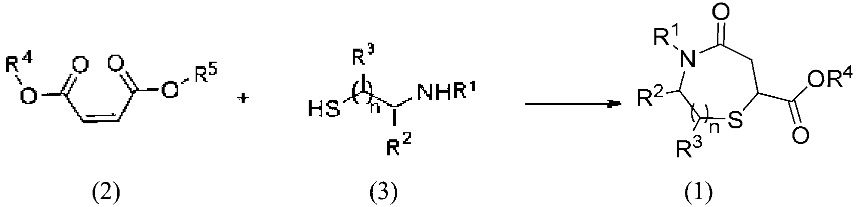 worin:
R¹, R⁴, R⁵ sind unabhängig voneinander ausgewählt aus der Gruppe umfassend Wasserstoff und C₁-C₃-Alkyl,
R², R³ sind unabhängig voneinander ausgewählt aus der Gruppe umfassend Wasserstoff, C₁-C₃-Alkyl, COOH und COOMe,
n ist 1 oder 2.

4. Verfahren nach Anspruch 3, **dadurch gekennzeichnet, dass** das Dialkylmaleat gemäß der allgemeinen Formel (2) ausgewählt ist aus der Gruppe umfassend Dimethylmaleat und Diethylmaleat.

5. Verfahren nach Anspruch 3 oder 4, **dadurch gekennzeichnet, dass** das Aminoalkylthiol gemäß der allgemeinen Formel (3) ausgewählt ist aus der Gruppe umfassend 2-Aminoethanthiol (Cysteamin) und 3-Aminopropanthiol.

6. Verfahren nach einem der vorhergehenden Ansprüche, **dadurch gekennzeichnet, dass** man die Umsetzung unter Zugabe von Triethylamin durchführt, vorzugsweise mit einem Überschuss an Triethylamin im Bereich von ≥ 1,2 eq. bis ≤ 1,5 eq..

7. Verfahren nach einem der vorhergehenden Ansprüche, **dadurch gekennzeichnet, dass** man die Umsetzung in einem Lösemittel ausgewählt aus der Gruppe umfassend Wasser, Alkohole, Dioxane, Acetonitril, Ether, Furan, Tetrahydrofuran, oder deren Gemischen durchführt.

8. Verfahren nach einem der vorhergehenden Ansprüche, **dadurch gekennzeichnet, dass** man die Umsetzung bei einer Temperatur im Bereich von ≥ 5 °C bis ≤ 95 °C, vorzugsweise bei einer Temperatur im Bereich von ≥ 15 °C bis ≤ 25 °C, durchführt.

9. Verfahren nach einem der vorhergehenden Ansprüche, **dadurch gekennzeichnet, dass** man das schwefelhaltige Lactam, insbesondere schwefelhaltige Caprolactam, mittels Extraktion mit Diethylether mit einer Temperatur im Bereich von ≥ 25 °C bis ≤ 40 °C aufreinigt.

10. Schwefelhaltiges Lactam nach Anspruch 1 oder 2, hergestellt nach dem Verfahren gemäß einem der Ansprüche 3 bis 9.
